# DEMANDE DE BREVET EUROPEEN

(11) **EP 0 572 742 A1**
(43) Date de publication de la demande: **08.12.1993**
(21) Numéro de dépôt: 92401567.0
(22) Date de dépôt: 05.06.1992
(51) Int. Cl.: A61N 1/32

(54) **Dispositif générateur d'impulsions électriques pour usages thérapeutiques**

(71) Demandeur: SOCIETE ANATOMIA - LA BOUTIQUE DU DOS, F-75009 Paris (FR)
(72) Inventeur: Kogan, Henry, F-75007 Paris (FR)
(74) Mandataire: Madeuf, René Louis

(57) **Abrégé**

Le dispositif générateur d'impulsions électriques pour usages thérapeutiques comprend un corps (1) renfermant un générateur de type piezo-électrique (15) et une tête (4) présentant au moins deux électrodes reliées respectivement aux bornes du générateur. La gâchette (16) d'actionnement du générateur piezo-électrique est reliée à des moyens de commande (10, 17) présentant une cadence d'actionnement réglable, de sorte que la fréquence des impulsions aux bornes des électrodes varie en dépendance de ladite cadence d'actionnement.

## Description

La présente invention concerne les dispositifs générateurs d'impulsions électriques pour usages thérapeutiques qui sont utilisés pour des traitements divers dans lesquels il apparaît utile que des décharges et/ou des effluves soient amenées à circuler dans certaines parties du corps d'un patient.

On connaît déjà des dispositifs à usages thérapeutiques dans lesquels on produit le courant d'origine piezoélectrique à partir d'un générateur actionné à la main. De tels dispositifs sont utilisés en particulier pour le traitement des douleurs musculaires, le courant étant amené à circuler entre des électrodes que comporte le dispostif.

La présente invention concerne des dispositifs générateurs de ce type dans lesquels les décharges et/ou effluves sont produites par un générateur de type piezo-électrique, tout en permettant de mieux contrôler la fréquence des décharges et/ou effluves le long de leur ligne d'application sur le corps d'un patient en fonction de la thérapeutique appliquée.

Conformément à l'invention, le dispostif générateur d'impulsions électriques pour usages thérapeutiques comprend, d'une part, un corps renfermant un générateur de type piezo-électrique et d'autre part, une tête présentant au moins deux électrodes reliées respectivement aux bornes du générateur piezo-électrique, et est caractérisé en ce qu'une gâchette d'actionnement du générateur piezo-électrique est reliée à des moyens de commande présentant une cadence d'actionnement réglable, de sorte que la fréquence des impulsions aux bornes des électrodes varie en dépendance de ladite cadence d'actionnement.

Diverses autres caractéristiques de l'invention ressortent d'ailleurs de la description détaillée qui suit.

Des formes de réalisation de l'objet de l'invention sont représentées, à titre d'exemples non limitatifs, aux dessins annexés.

La fig. 1 est une vue en partie arrachée du dispositif conforme à l'invention laissant apparaître en traits mixtes les éléments actifs du dispositif.

La fig. 2 est une vue de dessus de la fig. 1.

Les fig. 3a et 3b représentent respectivement une demi-coquille inférieure et une demi-coquille supérieure dont l'assemblage permet de former le corps du dispositif.

La fig. 4 est une vue de face de la tête du dispositif.

La fig. 5 est une vue de face d'une pièce de montage du dispositif.

Les fig. 6a et 6b présentent des détails de réalisation d'une came de commande du dispositif vue selon deux plans de coupe à 90° l'un de l'autre.

La fig. 7 est un bloc diagramme de la partie active du dispositif.

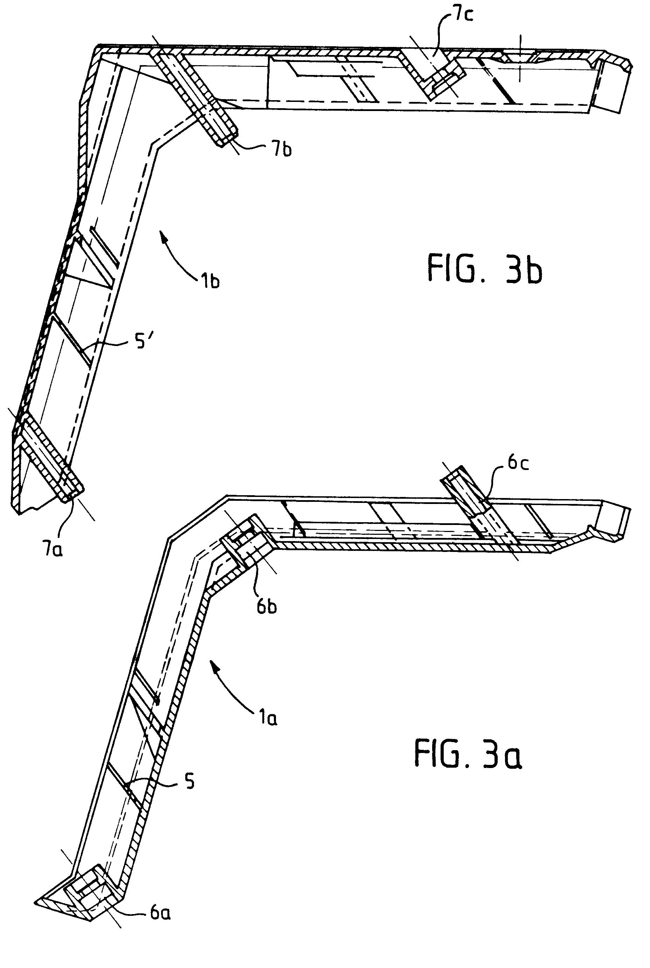

Le dispositif illustré au dessin comprend un corps 1 de forme quelconque mais qui est de préférence sensiblement en forme de pistolet et réalisé à partir de deux demi-coquilles la et 1b, respectivement représentées aux fig. 3a et 3b. Le corps délimite ainsi un bras 2 et un manche 3 dont les axes font entre eux un angle compris entre environ 100 et 110°. De préférence, cet angle est égal à 105°.

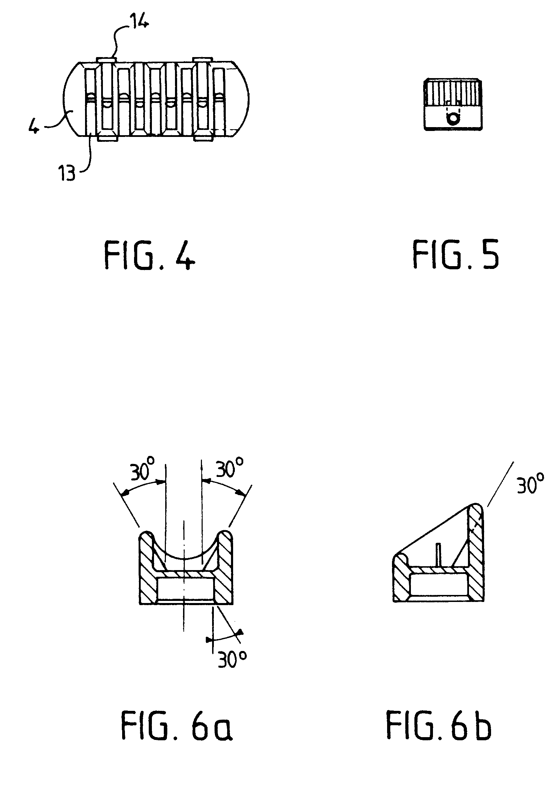

Une telle disposition angulaire du corps 1 permet au dispositif selon l'invention d'être facilement tenu à la main à l'aide du manche 3 par une personne devant appliquer une tête distributrice 4 (fig. 4) sur une partie de la peau à traiter, cette tête distributrice 4 étant disposée à l'extrémité libre du bras 2 du dispositif en étant inclinée de préférence d'environ 15° par rapport à l'axe du bras 2.

Comme on le voit à la fig. 3a, la demi-coquille inférieure la présente un certain nombre de nervures de raidissement telles que la nervure 5 et contient des logements 6a, 6b, 6c prévus pour coopérer avec des logements 7a, 7b, 7c correspondant sur la demi-coquille supérieure 1b illustrée à la fig. 3b et qui comporte également des nervures de raidissement 5'.

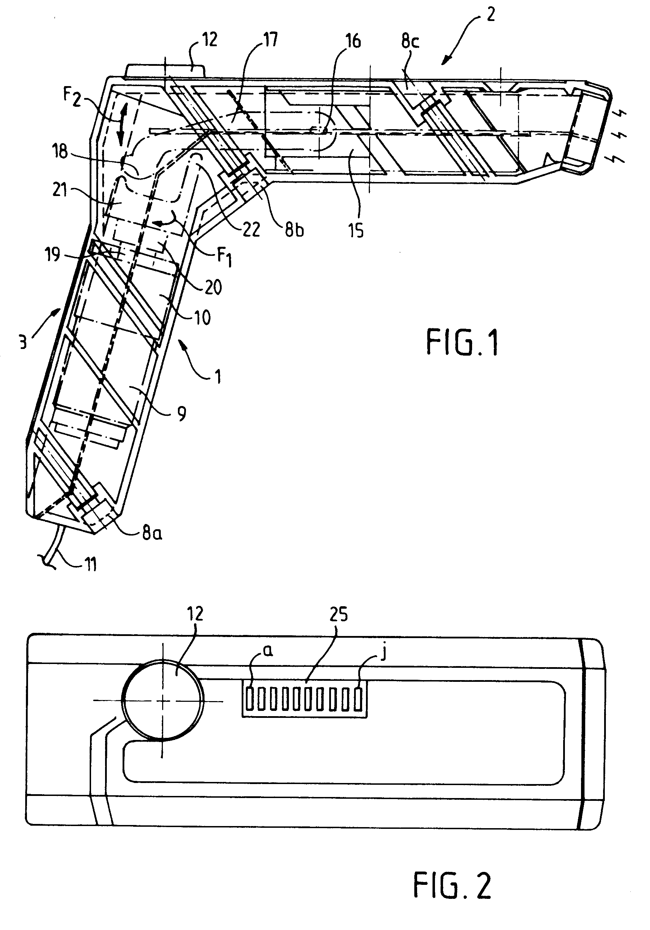

Les deux demi-coquilles la et 1b sont prévues pour s'emboîter l'une dans l'autre. Comme on le voit à la fig. 1, des vis 8a, 8b, 8c permettent une solidarisation des deux demi-coquilles la et 1b pour former le corps 1.

A la fig. 1, on a représenté en traits mixtes la partie active du dispositif placée à l'intérieur du corps 1.

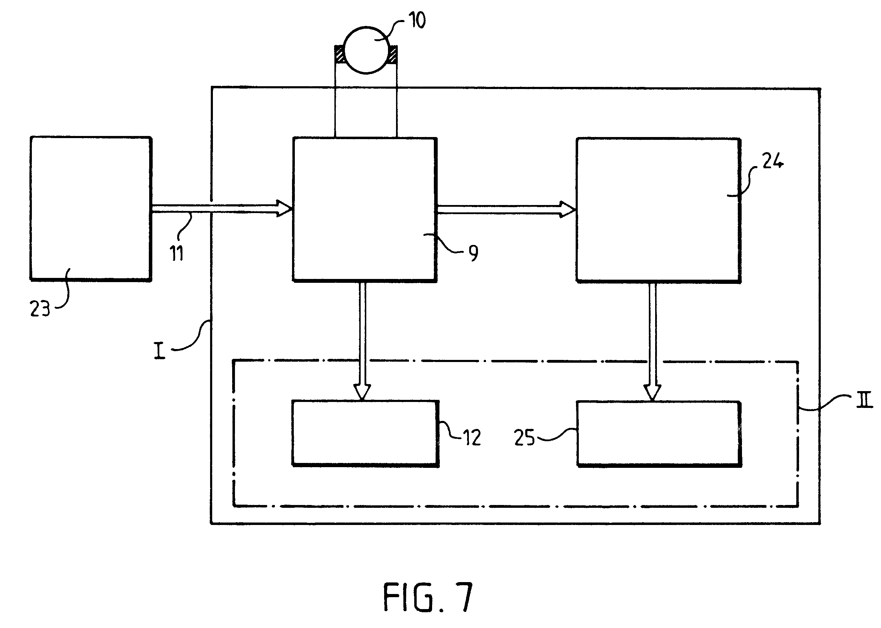

Un régulateur de tension 9, dont le but sera mieux compris à partir d'explications données plus loin en relation avec la fig. 7, est prévu pour alimenter un moto-réducteur 10.

Par ailleurs, le régulateur de tension 9 est relié par un fil 11, qui sort ainsi du corps 1 du dispositif, à un tranformateur de redressement et de filtrage non représenté à la fig. 1 et qui est alimenté par exemple à partir de la tension du secteur. Un tel bloc adaptateur est bien connu de la technique et n'a pas besoin d'être décrit plus en détail.

Un potentiomètre de réglage 12, comprenant un commutateur Marche/Arrêt, est monté sur le dessus du corps 1 et est relié au régulateur de tension 9 afin de faire varier la tension redressée et filtrée de sortie du régulateur de tension 9 et qui est appliquée au moto-réducteur 10.

Comme on le voit à la fig. 4, la tête distributrice 4 présente par exemple des électrodes 13, 14, alternativement de rang pair et de rang impair et qui sont respectivement reliées, en vue de leur alimentation en courant, aux bornes d'un générateur de type piezo-électrique 15 (fig. 1) monté dans le bras 2.

Conformément à l'invention, le générateur piezo-électrique 15 présente sa gâchette d'actionnement, dont l'extrémité sort en 16 du générateur, reliée à un levier de commande 17 dont l'extrémité libre de forme arrondie 18 est placée approximativement au point de rencontre des axes du bras 2 et de la poignée 3.

De même, le moto-réducteur 10 présente son arbre de sortie 19 relié par un insert métallique 20 (voir fig. 5) à une came 21 en matière plastique présentant une forme bien visible aux fig. 6a et 6b, et dont la partie supérieure 22 est également placée approximativement au point de rencontre des axes du bras 2 et de la poignée 3.

On voit par ce qui précède que la rotation du moto-réducteur 10 amène la came 21 à tourner dans le sens indiqué par la flèche F₁ et à entraîner selon un mouvement alternatif, illustré à la fig. 1 par la flèche F₂, de va-et-vient dans un plan vertical le levier de commande 17.

Ainsi par l'intermédiaire de son extrémité 16, la gâchette du générateur piezo-électrique 15 est actionnée selon une cadence dépendant de la vitesse de rotation du moto-réducteur 10, de sorte que des étincelles sont émises entre les électrodes 13 et 14 de la tête 4 avec une fréquence qui est elle-même fonction de la vitesse du moto-réducteur 10.

A la fig. 7, qui représente le bloc diagramme du dispositif de l'invention, les pièces des figures précédentes ont été indiquées par les mêmes références. En outre, on a figuré dans un rectangle en trait plein I et de manière schématique un circuit imprimé portant les organes se trouvant à l'intérieur du corps 1 du dispositif et, dans un rectangle en traits mixtes II, la partie du circuit qui est visible au-dessus du corps 1.

Comme on le voit à la fig. 7, le transformateur de redressement et de filtrage dort il a été question précédemment et qui est ici référencé 23 est relié au régulateur de tension 9, et ce dernier commande le moto-réducteur 10 par l'intermédiaire du potentiomètre 12.

Le régulateur de tension 9 est en outre relié de préférence par l'intermédiaire d'un comparateur de tension 24, à un indicateur de tension 25 placé sur le dessus du corps 1 (voir fig. 2). L'indicateur de tension 25 peut être de tout type approprié et, dans la forme de réalisation préférée de l'invention, l'indicateur de tension 25 est un indicateur de tension à diodes électroluminescentes appelé bar-graph dans la technique. Un tel indicateur de tension est constitué de dix diodes électroluminescentes (a ... j), généralement colorées en rouge, et dont la première s'allume lors de la mise en marche de l'appareil par l'intermédiaire du commutateur Marche/Arrêt du potentiomètre 12, les différentes diodes électroluminescentes s'allumant ensuite chacune à leur tour par pas de 1 volt lors de la manoeuvre du potentiomètre 12, en éteignant à chaque fois la précédente jusqu'à la dixième diode électroluminescente indiquant la tension maximale appliquée par le régulateur de tension au moto-réducteur 10. De préférence, le potentiomètre 12 est de type linéaire, c'est-à-dire que la tension à ses bornes varie de manière linéaire par une manoeuvre progressive de ce potentiomètre.

Le comparateur de tension 24 est un comparateur à échelle de type connu comprenant ici une série de dix échelons, et ce comparateur apprécie la valeur de la tension de sortie du régulateur 9 appliquée à l'entrée du moto-réducteur 11, cette tension étant visualisée par l'indicateur de tension 25.

On voit ainsi que, en fonction de la tension appliquée au moto-réducteur 11 qui, dans une forme préférée de l'invention peut varier de 12 à 22 Volts selon la position donnée par l'opérateur au potentiomètre linéaire 12, ce qui est apprécié par l'indicateur de tension 25, il est possible de commander de manière régulière la rotation du moto-réducteur qui s'effectue alors sans diminution du couple appliqué au bras de commande 17 du générateur piezo-électrique 15 par l'intermédiaire de la came 18, de manière à faire varier, de façon régulière, la fréquence du courant d'origine piezo-électrique sortant du générateur et appliqué entre les électrodes 13 et 14 de la tête 4. On peut donc aisément contrôler la fréquence des décharges et/ou effluves émises le long de leur ligne d'application sur le corps d'un patient en fonction de la thérapeutique appliquée.

L'invention n'est pas limitée aux formes de réalisation représentées et décrites en détail car diverses modifications peuvent y être apportées sans sortir de son cadre.

## Revendications

1. Dispositif générateur d'impulsions électriques pour usages thérapeutiques comprenant, d'une part, un corps (1) renfermant un générateur de type piezo-électrique (15) et, d'autre part, une tête (4) présentant au moins deux électrodes (13, 14) reliées respectivement aux bornes du générateur piezo-électrique, caractérisé en ce qu'une gâchette (16) d'actionnement du générateur piezo-électrique est reliée à des moyens de commande (10, 17) présentant une cadence d'actionnement réglable, de sorte que la fréquence des impulsions aux bornes des électrodes (13, 14) varie en dépendance de ladite cadence d'actionnement.

2. Dispositif selon la revendication 1, caractérisé en ce que les moyens de commande (10, 17) comportent un moto-réducteur (10) alimenté sous une tension réglable par l'intermédiaire d'un régulateur de tension (9) contrôlé par un potentiomètre (12).

3. Dispositif selon l'une des revendications 1 ou 2, caractérisé en ce qu'un indicateur de tension, tel qu'un indicateur à diodes électroluminescentes (25), est monté aux bornes du régulateur de tension (9).

4. Dispositif selon l'une des revendications 1 à 3, caractérisé en ce qu'un comparateur de tension (24) est monté entre l'indicateur (25) et le régulateur de tension (9).

5. Dispositif selon l'une des revendications 1 à 4. caractérisé en ce que l'arbre de sortie (19) du moto-réducteur (10) comporte une came (21) agissant sur un levier (17) faisant partie des moyens de commande (10, 17) de la gâchette (16) d'actionnement du générateur piezo-électrique (15) pour déplacer ce levier selon un mouvement alternatif de va-et-vient.

6. Dispositif selon l'une des revendications 1 à 5, caractérisé en ce que le corps (1) est réalisé à partir de deux demi-coquilles (1a, 1b) emboîtées, ce corps délimitant, d'une part, un manche (3) et, d'autre part, un bras (2) à l'extrémité duquel est montée la tête (4), l'axe du manche et l'axe du bras faisant un angle compris entre environ 100 et 110°, l'extrémité (18) du levier de commande, agissant sur la came (21), étant placée sensiblement au point de rencontre des deux axes.

7. Dispositif selon l'une des revendications 1 à 6, caractérisé en ce que l'angle que fait l'axe du manche (3) avec l'axe du bras (2) est sensiblement égal à 105°.

8. Dispositif selon l'une des revendications 1 à 7, caractérisé en ce que la tête (4) est inclinée d'environ 15° par rapport à l'axe du bras (2).

9. Dispositif selon l'une des revendications 1 à 8, caractérisé en ce que le régulateur de tension (9) est alimenté en courant à partir de la tension du secteur par l'intermédiaire d'un transformateur de redressement et de filtrage (23).

10. Dispositif selon l'une des revendications 1 à 9, caractérisé en ce que le potentiomètre de contrôle (12) du régulateur de tension (9) est du type linéaire.

11. Dispositif générateur d'impulsions électriques pour usages thérapeutiques sensiblement tel que décrit et représenté aux dessins annexés.
